(19) Europäisches Patentamt
European Patent Office
Office européen des brevets

(11) Publication number: **0 088 752 B1**

(12) **EUROPEAN PATENT SPECIFICATION**

(45) Date of publication of patent specification: **22.01.86**

(51) Int. Cl.[4]: **C 07 K 7/06,** A 61 K 37/00

(21) Application number: **81902712.9**

(22) Date of filing: **21.09.81**

(86) International application number: **PCT/US81/01271**

(87) International publication number: **WO 83/01062 31.03.83 Gazette 83/08**

# (54) ANTIGENIC PEPTIDE COMPOUND.

(43) Date of publication of application: **21.09.83 Bulletin 83/38**

(45) Publication of the grant of the patent: **22.01.86 Bulletin 86/04**

(84) Designated Contracting States: **DE FR GB LU NL SE**

(56) References cited:
**US-A-4 016 259**
**US-A-4 161 522**
**US-A-4 290 944**

**Biochemical and biophysical research communication, vol. 74, issued 1977, pages 1066-1070, Wheat et al.**
**The Journal of Biological Chemistry, vol. 247, issued 1972, pages 2044-2048, Goldberg**

(73) Proprietor: **NORTHWESTERN UNIVERSITY**
**Rebecca Crown Center**
**Evanston Illinois 60201 (US)**

(72) Inventor: **GOLDBERG, Erwin**
**2732 Hampton Parkway**
**Evanston, IL 60201 (US)**

(74) Representative: **Patentanwälte Grünecker, Dr. Kinkeldey, Dr. Stockmair, Dr. Schumann, Jakob, Dr. Bezold, Meister, Hilgers, Dr. Meyer-Plath**
**Maximilianstrasse 58**
**D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Background and prior art

Mammalian spermatozoa have been known to be antigenic for many years. More recently, it has been demonstrated that mammalian sperm contain an antigenic enzyme, which is known as the $C_4$ isozyme of lactate dehydrogenase (LDH—X, LDH—$C_4$). LDH—$C_4$ has been isolated in pure crystalline form from mouse testes. Goldberg (1972) *J. Biol. Chem.* 247:2044—2048. The enzyme has a molecular weight of 140,000 and is composed of four identical C subunits. The amino acid sequence and three-dimensional structure of LDH—$C_4$ has been studied and partially determined by a number of investigators. See Musick et al (1976) *J. Mol. Biol.* 104:659—668; and Wheat et al (1977) *Biochem. & Biophys. Res. Comm.*, 74 No. 3:1066—1077. Wheat et al determined the sequence of the essential thiol peptide from amino acid 159 to 171, and found this to be nearly identical to essential thiol peptides from other vertebrate LDH isozymes.

In 1974, Dr. Erwin Goldberg reviewed the effects of immunization with LDH—X (LDH—$C_4$) on fertility, and advanced the possibility that "by using a defined macromolecular constituent of sperm it becomes possible to elucidate its primary structure in terms of amino acid sequence, to map specifically the antigenic determinant(s) responsible for inducing infertility, and then to construct synthetic peptides containing these determinants. Possessing the capability for synthesizing a molecule with such properties, makes the immunological approach to fertility control feasible". Karolinska Symposia on Research Methods in Reproductive Encocrinology, 7th Symposia: Immunological Approaches to Fertility Control, Geneva, 1974 202—222. However, such synthetic antigenic peptides remained a goal and not an achievement although their theoretical desirability had been recognized. In 1979, Dr. Erwin Goldberg summarized the state of the art as follows:

"In conclusion, and on a practical basis immunotherapy for birth control requires more than effectiveness, specificity, reversibility and absence of systemic side reaction. Rather, large amounts of the antigen must be available in unequivocally pure form. This condition probably cannot be met by a natural product enzyme antigen from sperm or testes. Rather, contraceptive technology requires a synthesizable peptide fragment retaining antigenicity and provoking a response which impairs fertility. Completion of the structural analysis of LDH—$C_4$ should allow mapping of antigenic determinants and synthesis of such peptides for uses in a new contraceptive technology," "Recent Advances in Reproduction and Regulation of Fertility," G. P. Talwar, editor Elsevier/North Holland Biomedical Press (1979).

Summary of invention

It has now been discovered that an antigenic peptide can be prepared by synthesizing a linear sequence of seven amino acids comprising: methionine-glutaminelysine-aspartic acid-leucine-glutamic acid-leucine. All of the amino acids used to prepare the foregoing peptide are in their L-form. The N-terminal is methionine and the C-terminal is leucine. Although not known with certainty, it is believed that the foregoing sequence of seven amino acids corresponds to amino acids 325 to 332 of LDH—$C_4$. This is contrary to a recently published tentative sequence. See Musick et al (1979) *J. Biol. Chem.*, 254, No. 16:7621—7623.

Description of invention

The present invention relates to a novel antigenic linear peptide having a chain length of seven amino acids. The formula of this peptide can be represented as follows:

N-Met-Gln-Lys-Asp-Leu-Glu-Leu-C

In the foregoing formula, the letter "N" designates the N-terminal amino acid, while the letter "C" designates the C-terminal amino acid. Met, Gln, Lys, Asp, Glu, and Leu represent the L-amino acid forms, respectively, of methionine, glutamine, lysine, aspartic acid, glutamic acid, and leucine.

The peptide compound of the present invention can be synthesized from its constituent amino acids. For example, the synthesis can be carried out by the Merrifield solid phase method, as described in *J.A.C.S.* 85:2149—2154 (1963). This solid phase method for synthesizing sequences of amino acids is also described in Stewart and Young, *Solid Phase Peptide Synthesis* (W. H. Freeman and Co., San Francisco, 1969), pages 1—4. In this procedure, the C-terminal amino acid, such as leucine for the compound of this invention, is attached to chloromethylated polystyrene-divinylbenzene copolymer beads. Each subsequent amino acid, with suitable protecting group, is then added sequentially to the growing chain. For example, as described in the Merrifield article, the protective group may be a carbobenzoxy group. By the procedure of coupling, deprotection, and coupling of the next amino acid, the desired amino acid sequence and chain length can be produced. As a final step, the protective group is removed from the N-terminal amino acid (viz. methionine), and the C-terminal amino acid is cleaved from the resin, using a suitable reagent, such as trifluoroacetic acid and hydrogen bromide. Since this synthesis procedure is well known, it is not believed that it will be necessary to further describe it herein. The peptide of this invention can be prepared by this synthesis procedure for use in reducing the fertility of mammals.

To utilize the antigenic peptide of this invention in the form of a fertility reducing vaccine, the peptide is conjugated to a carrier molecule, which is preferably a protein which itself elicits an antigenic response and which can be safely administered. For example, the peptide can be

coupled to tetanus toxoid for administration by intramuscular injection. For example, a mixture of 1μ Mole tetanus toxoid 60 μ Moles antigenic peptide and 18 millimoles 1-ethyl-3-(3 dimethyl aminopropyl) carbodiimide hydrochloride reacted in water (pH 6) for 12 hours at room temperature and 24 hours at 4°C gives a product containing 3.5 moles of peptide/mole tetanus toxoid. Excess reactants can be removed by dialysis or gel filtration. See Pique et al, *Immunochemistry*, 15:55—60 (1978). Alternatively, the peptide may be coupled using bisdiazotized benzidine [Bassiri et al, *Endocrinology*, 90:722 (1972)] or glutaraldehyde.

For intramuscular injection, the coupled peptide may be suspended in a sterile isotonic saline solution, or other conventional vehicle, and, if desired, an adjuvant may be included. A preferred use of such a vaccine is for administration to human females. Antibodies will be formed, which will appear in the oviduct fluids and thereby achieve a significant reduction in fertility. For this purpose, the amount to be administered will range from about 1 to 10 milligrams (mg) of the antigenic peptide.

**Claim**

The antigenic linear peptide compound having the formula:

N-Met-Gln-Lys-Asp-Leu-Glu-Leu-C

wherein N-Met is N-terminal L-methionine, Leu-C is C-terminal L-leucine, and Gln, Lys, Asp, Glu, and Leu are the L-amino acid forms, respectively, of glutamine, lysine, aspartic acid, glutamic acid, and leucine.

**Revendications**

Composé de peptide linéaire antigénique répondant à la formule:

N-Met-Gln-Lys-Asp-Leu-Glu-Leu-C

dans laquelle N-Met est la L-méthionine N-terminale, Leu-C est la L-leucine C-terminale et Gln, Lys, Asp, Glu et Leu sont respectivement les formes L des acides aminés glutamine, lysine, acide aspartique, acide glutamique et leucine.

**Patentanspruch**

Antigene, lineare Peptidverbindung mit der Formel

N-Met-Gln-Lys-Asp-Leu-Glu-Leu-C,

worin N-met L-Methionin mit endständigem N, Leu-C L-Leucin mit endständigem C, und Gln, Lys, Asp, Glu und Leu die L-Aminosäureformen von Glutamin, Lysin, Asparaginsäure Glutaminsäure bzw. Leucin sind.